(19) Europäisches Patentamt
European Patent Office
Office européen des brevets

(11) Numéro de publication: 0 223 648 B1

# (12) FASCICULE DE BREVET EUROPEEN

(45) Date de publication du fascicule du brevet: 20.12.89

(51) Int. Cl.[4]: C07C 127/19, C07C 143/74, C07C 155/02, C08F 20/36, C14C 11/00

(21) Numéro de dépôt: 86402225.6

(22) Date de dépôt: 07.10.86

(54) **Monomères acryliques fluorés, polymères en dérivant et leur application au traitement hydrofuge et oléofuge de substrats divers.**

(30) Priorité: 16.10.85 FR 8515347

(43) Date de publication de la demande: 27.05.87 Bulletin 87/22

(45) Mention de la délivrance du brevet: 20.12.89 Bulletin 89/51

(84) Etats contractants désignés: BE CH DE FR GB IT LI

(56) Documents cités: FR-A-2 144 204

(73) Titulaire: ATOCHEM, 4 & 8, Cours Michelet La Défense 10, F-92800 Puteaux(FR)

(72) Inventeur: Lina, Marie-José, Résidence "Les Jonquières" 6, rue Boyer, F-69160 Tassin La Demi Lune(FR)
Inventeur: Dessaint, André, 17 B, rue des Racques, Boulincourt F-60600 Clermont(FR)

(74) Mandataire: Leboulenger, Jean et al, ATOCHEM Département Propriété Industrielle, F-92091 Paris la Défense 10 Cédex 42(FR)

EP 0 223 648 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine des produits fluorés destinés au traitement hydrofuge et oléofuge de substrats tels que textiles, tapis-moquettes, revêtements muraux, bois, matériaux de construction, métaux, matières plastiques, et a notamment pour objet des produits utilisables plus particulièrement pour la protection du cuir dont la finition et l'entretien doivent présenter les caractéristiques suivantes : souplesse, aspect et toucher agréables.

L'utilisation de résines acryliques fluorées dans ces applications est bien connue, mais présente un certain nombre d'inconvénients : toucher légèrement collant, mauvaise résistance aux nettoyages et à l'abrasion, légère modification de l'aspect du support.

Des compositions comprenant des groupes perfluorés et des enchaînements uréthannes ont déjà été proposées ; voir par exemple les brevets U.S. 3 468 924, 3 503 915, 3 528 849, 3 896 035, 3 896 251 et 4 024 178, FR 2 062 244, DE 1620 965, CA 1 071 225, EP 103752, CH 520 813 et 512 624. Malheureusement, ces produits ne donnent pas toujours satisfaction soit parce que la synthèse des intermédiaires est difficile, soit parce qu'ils doivent être associés à des copolymères acryliques car ils ne sont pas filmogènes, ne résistent pas au nettoyage à sec et/ou ne présentent pas de bonnes propriétés antitaches, soit encore parce qu'ils doivent être présentés en émulsion aqueuse en raison de leur faible solubilité dans les solvants.

Il a maintenant été trouvé dans les services de la demanderesse une nouvelle classe de monomères acryliques fluorés dont les polymères présentent d'excellentes propriétés hydrophobes et oléophobes et sont, en raison des propriétés mécaniques de leurs films (adhérence au support, transparence, résistance à l'abrasion), parfaitement adaptés au traitement du cuir.

Les monomères acryliques fluorés selon la présente invention qui portent à la fois une liaison uréthanne et une liaison urée ou deux liaisons urée, peuvent être représentés par la formule générale:

$$R_F-W-Q-\underset{\underset{O}{\|}}{C}-NH-Z-NH-\underset{\underset{O}{\|}}{C}-\overset{\overset{R'}{|}}{N}-A-O-\underset{\underset{O}{\|}}{C}-\overset{\overset{R}{|}}{C}=CH_2 \qquad (I)$$

dans laquelle:

$R_F$ représente un radical perfluoroalkyle à chaîne droite ou ramifiée,

R représente un atome d'hydrogène ou un radical méthyle,

R' représente un radical alkyle ou cycloalkyle ou –NR'– représente un radical pipérazinylène-1,4,

W–Q représente un enchaînement bivalent choisi parmi ceux de formules:

$$-(CH_2)_p-O-$$
$$-SO_2\underset{R''}{N}-(CH_2)_q-O-$$
$$-(CH_2)_p-SO_2\underset{R''}{N}-(CH_2)_q-O-$$
$$-(CH_2)_p-O-(CH_2)_q-O-$$
$$-(CH_2)_p-S-(CH_2)_q-O-$$
$$-(CH_2)_p-(OCH_2CH_2)_q-O-$$
$$-(CH_2)_p-SO_2-(CH_2)_q-O-$$
$$-\underset{O}{\overset{}{\underset{\|}{C}}}-\underset{R''}{N}-(CH_2)_p-O-$$
$$-\underset{O}{\underset{\|}{C}}-O-(CH_2)_p-O-$$
$$-CH=CH-(CH_2)_p-O-$$

$$-(CH_2)_pS-$$
$$-(CH_2)_pNH-$$
$$-SO_2-\underset{R''}{N}-(CH_2)_qNH-$$

$$-SO_2-\underset{R''}{N}-(CH_2)_q-\underset{CH_3}{N}-$$

R″ désigne un atome d'hydrogène ou un radical alkyle,
p et q, identiques ou différents, représentent chacun un nombre entier allant de 1 à 20, et de préférence égal à 2 ou 4,
Z représente un enchaînement bivalent aliphatique, cycloaliphatique ou aromatique, et
A représente un groupe alkylène de 2 ou 3 atomes de carbone.

Le radical perfluoroalkyle $R_F$ peut contenir de 2 à 20 atomes de carbone. On préfère cependant les composés dans lesquels $R_F$ est un radical perfluoroalkyle linéaire contenant 6, 8 ou 10 atomes de carbone.

Le radical alkyle R′ peut être à chaîne droite ou ramifiée et contenir de 1 à 12 atomes de carbone. On préfère les composés dans lesquels R′ contient 2 à 4 atomes de carbone et plus particulièrement ceux dans lesquels R′ est le radical tertiobutyle.

Les monomères acryliques fluorés de formule (I) selon l'invention peuvent être préparés en faisant réagir sur un diisocyanate de formule:

$$O=C=N-Z-N=C=O \quad (II)$$

des quantités sensiblement équimolaires d'un composé polyfluoré de formule :

$$R_F-W-Q-H \quad (III)$$

et d'un ester acrylique de formule :

$$H-\underset{\displaystyle R'}{\underset{|}{N}}-A-O-\underset{\displaystyle O}{\underset{\|}{C}}-\overset{\displaystyle R}{\overset{|}{C}}=CH_2 \qquad (IV)$$

Comme exemples de diisocyanates utilisables, on peut citer des diisocyanates aromatiques tels que le toluène-diisocyanate-2,4 et/ou -2,6, le diphénylméthane- diisocyanate-4,4′ et le xylylène-diisocyanate, des diisocyanates aliphatiques tels que l'hexaméthylènediisocyanate et le décaméthylènediisocyanate, et des diisocyanates cycloaliphatiques tels que le méthylène-bis(cyclohexylisocyanate)-4,4′, le méthyl-2 cyclohexane-diisocyanate-1,4 et l'isophoronediisocyanate. Parmi ces diisocyanates, on préfère le toluène-diisocyanate-2,4 seul ou en mélange avec l'isomère-2,6.

Le composé polyfluoré (III) est un composé contenant un atome d'hydrogène mobile sous forme d'un groupement terminal hydroxyle, thiol ou amino primaire ou secondaire, lié au radical perfluoroalkyle par un pont alkylène directement ou via un groupement sulfonamido, carbonamido, éther, thioéther, sulfonyle ou ester carboxylique. Comme exemples de tels composés polyfluorés, on peut citer plus particulièrement ceux de formules:

$$R_F-(CH_2)_p-OH \qquad (III-a)$$

$$R_F-SO_2\underset{\displaystyle R''}{\underset{|}{N}}-(CH_2)_q-OH \qquad (III-b)$$

$$R_F-(CH_2)_p-SO_2\underset{\displaystyle R''}{\underset{|}{N}}-(CH_2)_q-OH \qquad (III-c)$$

$$R_F-(CH_2)_p-O-(CH_2)_q-OH \qquad (III-d)$$

$$R_F-(CH_2)_p-S-(CH_2)_q-OH \qquad (III-e)$$

$$R_F-(CH_2)_p-(OCH_2\,CH_2)_q-OH \qquad (III-f)$$

$$R_F-(CH_2)_p-SO_2-(CH_2)_q-OH \qquad (III-g)$$

$$R_F-\underset{\displaystyle O}{\underset{\|}{C}}-\overset{\displaystyle R''}{\overset{|}{N}}-(CH_2)_p-OH \qquad (III-h)$$

$$R_F-\underset{\displaystyle O}{\underset{\|}{C}}-O-(CH_2)_p-OH \qquad (III-i)$$

$$R_F-CH=CH-(CH_2)_p-OH \qquad (III-j)$$

$$R_F-(CH_2)_pSH \qquad (III-k)$$

$$R_F-(CH_2)_pNH_2 \qquad (III-l)$$

$$R_F-SO_2-\underset{\displaystyle R''}{\underset{|}{N}}-(CH_2)_qNH_2 \qquad (III-m)$$

$$R_F-SO_2-\underset{\displaystyle R''}{\underset{|}{N}}-(CH_2)_q-N\begin{matrix}\diagup CH_3\\ \diagdown H\end{matrix} \qquad (III-n)$$

dans lesquelles $R_F$, R″, p et q ont les mêmes significations que ci-dessus. Pour des raisons économiques et pratiques, il est particulièrement intéressant d'utiliser un mélange de composés représentant des radicaux $R_F$ différents.

Parmi les composés (III) on préfère ceux de formules (III-a), (III-c), (III-k) dans lesquels p et q sont égaux à 2.

Comme exemples d'esters de formule (IV), on peut citer plus particulièrement les esters acryliques et méthacryliques du t-butyl-amino-2 éthanol, du t-octylamino-2 éthanol, du cyclohexylamino-2 éthanol, du laurylamino-2 éthanol et du pipérazino-2 éthanol. Un composé de formule (IV) préféré est le méthacrylate de t-butylamino-2 éthyle de formule:

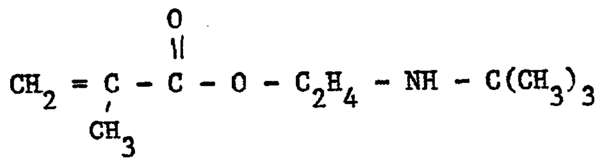
(IV-a)

La synthèse des monomères acryliques fluorés (I) selon l'invention peut être effectuée dans un solvant organique tel que les solvants cétoniques comme la méthyl éthyl cétone ou la méthylisobutyl cétone, des esters comme l'acétate d'éthyle ou l'acétate de butyle, des solvants aromatiques comme le toluène, des alcanes comme l'hexane, l'heptane ou le cyclohexane, des éthers comme le diisopropyl éther ou le tétrahydrofuranne, des solvants halogénés comme le trichloro-1,1,1 éthane ou le trichlorotrifluoroéthane, ainsi que le diméthyl formamide et la N-méthyl-pyrrolidone.

Les réactions d'addition du composé polyfluoré $R_F$–W–Q–H et de l'ester acrylique (IV) sur les groupements –N=C=O s'effectuent entre 30 et 90°C sous atmosphère inerte, par exemple sous azote anhydre. L'addition du composé polyfluoré étant lente, il est préférable d'opérer en présence d'un catalyseur tel que, par exemple, une amine tertiaire comme la triéthylamine, la triéthylènediamine et la N-méthyl-morpholine, un sel d'étain comme le dibutyl dilaurate d'étain et l'octoate d'étain, ou un sel de plomb comme le naphténate de plomb, ce catalyseur étant utilisé à raison de 0,05 à 1% par rapport au poids total des réactifs.

Afin de limiter la formation de produits de diadditions symétriques, on ajoute le composé polyfluoré $R_F$–W–QH goutte à goutte, dans des conditions de dilution et de température telles que la réaction soit quasi instantannée et que l'on soit toujours en défaut de $R_F$ W QH par rapport à l'isocyanate. L'ester acrylique porteur de groupe –NH plus réactif, est ajouté dans un deuxième temps. Il réagit très facilement avec les N=C=O restés libres. On ne peut pas éviter complètement la formation de composés de diadditions symétriques; mais il est possible, si on le désire, de les éliminer par filtration, compte-tenu de leur solubilité moindre dans les solvants réactionnels que les monomères uréthanne/urée de formule (I).

L'invention a également pour objet les polymères contenant de 10 à 100% en poids de motifs de formule:

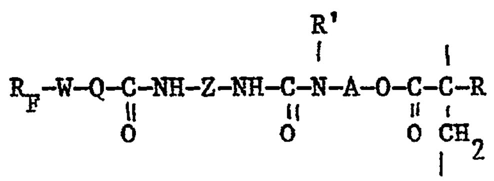
(V)

dans laquelle les différents symboles ont les mêmes significations que précédemment. Ces polymères peuvent être obtenus à partir des monomères de formule (I) par homopolymérisation ou par copolymérisation avec d'autres monomères, fluorés ou non, en une proportion allant jusqu'à 90 % en poids par rapport au poids total de monomères.

Comme exemples de comonomères utilisables dans le cadre de la présente invention, on peut citer :
- les hydrocarbures oléfiniques inférieurs, halogénés ou non, tels que l'éthylène, le propylène, l'isobutène, le chloro-3 isobutène-1, le butadiène, l'isoprène, les chloro- et dichloro-butadiènes, les fluoro- et difluoro-butadiènes, le diméthyl-2,5 hexadiène-1,5, le diisobutylène ;
- les halogénures de vinyle, d'allyle ou de vinylidène tels que le chlorure de vinyle ou de vinylidène, le fluorure de vinyle ou de vinylidène, le bromure d'allyle, le chlorure de méthallyle ;
- le styrène et ses dérivés, tels que le vinyl-toluène, l'α-méthyl-styrène, l'α-cyanométhyl-styrène, le divinyl-benzène, le N-vinyl carbazole ;
- les esters vinyliques tels que l'acétate de vinyle, le propionate de vinyle, les esters vinyliques des acides connus sur le marché sous le nom de "Versatic acids", l'isobutyrate de vinyle, le sénécioate de vinyle, le succinate de vinyle, l'isodécanoate de vinyle, le stéarate de vinyle, le carbonate de divinyle ;
- les esters d'allyle comme l'acétate d'allyle et l'heptanoate d'allyle ;
- les éthers alkyl-vinyliques ou alkyl-allyliques, halogénés ou non, tels que le cétyl vinyl éther, le dodécyl vinyl éther, l'isobutyl vinyl éther, l'éthyl vinyl éther, le chloro-2 éthyl vinyl éther, le tétra allyloxy éthane ;
- les vinyl alkyl cétones comme la vinyl méthyl cétone ;
- les acides insaturés, tels que les acides acrylique, méthacrylique, α-chloro-acrylique, crotonique, maléique, fumarique, itaconique, citraconique et sénécioïque, leurs anhydrides et leurs esters comme les acrylates et méthacrylates de vinyle, d'allyle, de méthyle, de butyle, d'isobutyle, d'hexyle, d'heptyle, d'éthyl-2 hexyle, de cyclohexyle, de lauryle, de stéaryle ou de cellosolve, le maléate de diméthyle, le crotonate d'éthyle, le maléate acide de méthyle, l'itaconate acide de butyle, les diacrylates et diméthacryla-

tes de glycol ou de polyalkylène glycol, comme le diméthacrylate d'éthylène glycol ou de triéthylène glycol, les acrylates et méthacrylates de dichloro-phosphato alkyle comme le méthacrylate de dichlorophosphato éthyle, ainsi que le phosphate acide de bis (méthacryloyloxy éthyle) et le méthacryloyloxypropyl-triméthoxy-silane;
– l'acrylonitrile, le méthacrylonitrile, le chloro-2 acrylonitrile, l'acrylate de cyano-2 éthyle, le méthylène glutaronitrile, le cyanure de vinylidène, les cyanoacrylates d'alkyle comme le cyanoacrylate d'isopropyle, la trisacryloyl hexahydro-s-triazine, le vinyl trichlorosilane, le vinyl triméthoxysilane, le vinyl triéthoxysilane, la N-vinyl-pyrrolidone-2;
– l'alcool allylique, l'allyl glycolate, l'isobutènediol, l'allyloxy éthanol, l'o. allyl phénol, le divinyl carbinol, le glycérol-allyléther, l'acrylamide, le méthacrylamide, les maléamide et maléïmide, le N-(cyano-éthyl)-acrylamide, le N-isopropyl-acrylamide, le diacétone-acrylamide, les N-(hydroxy-méthyl) acrylamide et méthacrylamide, les N-(alcoxyméthyl) acrylamides et méthacrylamides, le glyoxal bis-acrylamide, l'acrylate ou méthacrylate de sodium, les acides vinyl-sulfonique et styrène-p-sulfonique et leurs sels alcalins, l'amino-3 crotononitrile, la monoallyl amine, les vinyl-pyridines, l'acrylate ou méthacrylate de glycidyle, l'allyl glycidyl éther, l'acroléine, le méthacrylate de N,N-diméthylaminoéthyle ou de N-tertio-butylaminoéthyle;
– les esters fluorés insaturés de formule générale:

$$R_F - W - O - \underset{\underset{O}{\parallel}}{C} - \overset{\overset{R}{|}}{C} = CH - R \qquad (VI)$$

dans laquelle W représente un enchaînement bivalent choisi parmi ceux de formules:

$$-(CH_2)_p-$$
$$-SO_2\underset{\underset{R''}{|}}{N}-(CH_2)_q-$$
$$-(CH_2)_p-SO_2\underset{\underset{R''}{|}}{N}-(CH_2)_q-$$
$$-(CH_2)_p-O-(CH_2)_q-$$
$$-(CH_2)_p-S-(CH_2)_q-$$
$$-(CH_2)_p-(OCH_2CH_2)_q-$$
$$-(CH_2)_p-SO_2-(CH_2)_q-$$
$$-\underset{\underset{O}{\parallel}}{C}-\underset{\underset{R''}{|}}{N}-(CH_2)_p-$$
$$-\underset{\underset{O}{\parallel}}{C}-O-(CH_2)_p-$$
$$-CH=CH-(CH_2)_p-$$

les symboles $R_F$, R, R", p et q ayant les mêmes significations que précédemment. Ces comonomères peuvent être préparés selon des procédés connus, par exemple par esterification des alcools polyfluorés correspondants de formule:

$$R_F - W - OH \qquad (VII)$$

au moyen d'un acide alcène-monocarboxylique de formule:

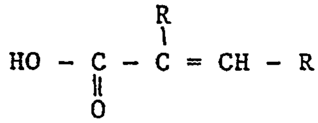

(VIII)

tel que, par exemple, l'acide acrylique, l'acide méthacrylique ou l'acide crotonique, en présence d'un catalyseur comme l'acide sulfurique ou l'acide p-toluènesulfonique. Au lieu des acides de formule (VIII), on peut également utiliser leurs esters, anhydrides ou halogénures. Comme exemples d'alcools polyfluorés de formule (VII), on peut citer plus particulièrement ceux de formules (III) a) à (III j).

A titre de comonomères utilisables dans le cadre de la présente invention, on peut encore mentionner:

- les esters insaturés de formule :

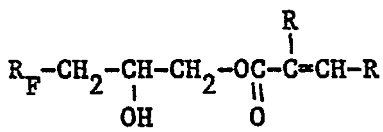

(IX)

obtenus par condensation d'un époxyde fluoré :

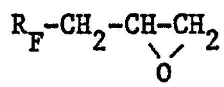

(X)

sur un acide alcène-monocarboxylique de formule (VIII); et les acrylates et méthacrylates d'éthers de polyéthylène glyclols ou de polypropylène glycols de formule :

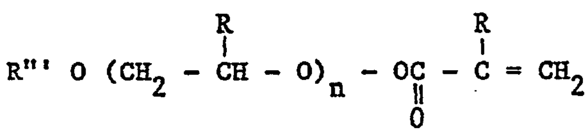

(XI)

dans laquelle R représente un atome d'hydrogène ou un radical méthyle, R" [1] représente un radical alkyle et n est un nombre entier compris entre 2 et 10.

Les polymères fluorés selon l'invention peuvent être préparés de façon connue en soi par polymérisation dans un solvant organique ou en émulsion aqueuse, à une température qui peut aller de la température ambiante jusqu'au point d'ébullition du milieu réactionnel. On opère de préférence entre 70 et 110°C. La concentration totale des monomères peut varier de 5 à 60 % en poids.

La polymérisation en milieu solvant peut être réalisée dans des solvants cétoniques (par exemple l'acétone, la méthyl éthyl cétone, la méthyl isobutyl cétone), des alcools (par exemple l'isopropanol), des esters (par exemple l'acétate d'éthyle ou l'acétate de butyle), des éthers (par exemple le diisopropyl éther, l'éther éthylique ou méthylique de l'éthylène glycol, le tétrahydrofuranne, le dioxanne), des hydrocarbures aliphatiques ou aromatiques, des hydrocarbures halogénés (par exemple le perchloréthylène, le trichloro-1,1,1 éthane, le trichlorotrifluoroéthane), le diméthylformamide ou la N-méthyl-pyrrolidone-2.

On effectue la polymérisation en présence d'initiateur(s) qu'on utilise à raison de 0,1 à 1,5 % par rapport au poids total des monomères engagés. Comme initiateurs on peut utiliser des peroxydes tels que, par exemple, le peroxyde de benzoyle, le peroxyde de lauroyle, le peroxyde de succinyle et le perpivalate de tertiobutyle, ou des composés azoïques tels que, par exemple, l'azo-2,2' bis-isobutyronitrile, l'azo-4,4' bis-(cyano-4 pentanoïque) et l'azodicarbonamide. On peut également opérer en présence de rayonnements U.V. et de photoinitiateurs tels que la benzophénone, la méthyl-2 anthraquinone ou le chloro-2 thioxanthone. La longueur des chaînes polymériques peut, si on le désire, être réglée à l'aide d'agents de transfert de chaînes tels que les alkyl-mercaptans, le tétrachlorure de carbone ou le triphénylméthane, utilisés à raison de 0,05 à 0,5 % par rapport au poids total de monomères.

La polymérisation en émulsion aqueuse peut être réalisée selon les techniques bien connues, en discontinu ou en continu. Les agents tensio-actifs à utiliser pour la mise en émulsion peuvent être cationiques, anioniques ou non-ioniques selon l'ionicité désirée pour le latex final, et sont de préférence choisis parmi les meilleurs émulsionnants huile-dans-eau, les moins mouillants possibles. On utilise de préférence des couples de tensio-actifs cationique / non-ionique ou anionique/non-ionique. Comme exemples d'agents tensio-actifs utilisables on peut citer plus particulièrement :

- dans la série cationique, les sels d'amines tertiaires à longue chaîne comme l'acétate de N,N-diméthyl octadécylamine, et les sels d'ammonium quaternaires d'amines grasses comme le bromure de triméthyl cétyl ammonium ou le chlorure de triméthyl dodécyl ammonium ;
- dans la série anionique, les sels alcalins d'acides alkylsulfoniques à longue chaîne et les arylalkylsulfonates alcalins ;

- dans la série non-ionique, les produits de condensation de l'oxyde d'éthylène avec les alcools gras ou les alkylphénols.

Il peut également être interessant d'utiliser des agents tensio-actifs à chaîne hydrophobe perfluorée tels que, par exemple, le perfluorooctanoate d'ammonium ou le N-perfluorooctyl-sulfonyl N-éthyl amino-acétate de potassium.

Pour faciliter la mise en émulsion des monomères, il est généralement nécessaire d'utiliser des solvants organiques tels que, par exemple, des cétones (acétone, méthyl éthyl cétone, méthyl isobutyl cétone), des glycols ou éthers d'éthylèneglycol, des alcools (méthanol, éthanol, isopropanol), ou des mélanges de ces solvants. La quantité de solvant ne doit généralement pas dépasser le poids total des monomères.

Comme initiateurs de polymérisation en émulsion aqueuse, on peut utiliser des produits solubles dans l'eau tels que les peroxydes minéraux (par exemple l'eau oxygénée) et les persels (par exemple le persulfate de potassium), ou des initiateurs insolubles dans l'eau comme les peroxydes organiques et les composés azoïques mentionnés précédemment.

Les polymères fluorés selon l'invention peuvent également être préparés par greffage d'un isocyanate-uréthanne fluoré de formule

$$R_F-W-Q-\underset{\underset{O}{\|}}{C}-NH-Z-N=C=O \qquad (XII)$$

sur un polymère acrylique à groupes >NH pendants, obtenu par homopolymérisation d'un ester acrylique de formule (IV) ou par copolymérisation d'un tel ester avec un ou plusieurs des comonomères précédemment cités. Les isocyanates-uréthannes de formule (XII) peuvent être préparés de la même façon et dans les mêmes conditions que les monomeres de formule (I) en faisant réagir un diisocyanate (II) avec une quantité sensiblement équimolaire d'un composé polyfluoré (III). Ces conditions opératoires valent également pour le greffage de l'isocyanate-uréthanne (XII)sur le polymère acrylique à groupes >NH pendants. Ce polymère peut lui-même être obtenu par polymérisation en milieu solvant dans des conditions analogues à celles précédemment décrites pour la polymérisation des monomères de formule (I).

Quelque soit leur mode d'obtention, les polymères fluorés selon l'invention peuvent éventuellement être isolés suivant des méthodes connues en soi telles que, par exemple, précipitation ou évaporation du solvant.

Les polymères fluorés selon l'invention se révèlent d'excellents agents hydrophobes et oléophobes sur des matériaux très divers tels que le papier, les articles non tissés, les textiles à base de fibres naturelles, artificielles ou synthétiques, les matières plastiques, le bois, les métaux, le verre, la pierre et le ciment, mais sont plus particulièrement destinés à la protection des cuirs qu'il s'agisse de leur finition ou de l'entretien des articles en cuir tels que vêtements, chaussures, maroquinerie, sièges ...

Pour l'application, les solutions de polymères sont généralement diluées avec un solvant identique à ou compatible avec celui utilisé pour la polymérisation, tandis que les émulsions de polymères sont diluées à l'eau. L'application des produits dilués peut être réalisée selon différentes techniques comme la pulvérisation, l'enduction à la brosse, le foulardage. Selon leur nature, les substrats traités peuvent être séchés à température ambiante ou à des températures pouvant aller jusqu'à 200°C.

La quantité de polymère à mettre en oeuvre peut varier dans de larges limites en fonction de la nature du support et de la teneur en fluor du polymère. Sur cuir, cette quantité est généralement comprise entre 1 et 10 g/m$^2$.

Les exemples suivants dans lesquels les parties et les pourcentages s'entendent en poids, sauf mention contraire, illustrent l'invention sans la limiter.

EXEMPLE 1

Dans un réacteur d'une capacité de 1000 parties en volume, équipé d'un agitateur, d'un thermomètre, d'un réfrigérant à reflux, d'une ampoule de coulée, d'une arrivée d'azote et d'un dispositif de chauffage on charge 370 parties de méthyl isobutyl cétone anhydre et 69,6 parties (0,4 mole) de toluène-diisocyanate (mélange à 80 % d'isomère-2,4 et 20 % d'isomère-2,6) et 0,4 partie de dibutyldilaurate d'étain. On chasse l'air du réacteur par un courant d'azote anhydre, puis on porte la température du milieu réactionnel à 80°C à l'aide d'un bain d'huile thermostaté et on coule ensuite goutte à goutte en une heure, une solution préalablement déshydratée par distillation, comprenant 145,6 parties de perfluorohexyl-éthanol $C_6F_{13}C_2H_4OH$ (soit 0,4 mole) et 145,6 parties de méthyl isobutyl cétone. Le mélange est encore maintenu sous agitation à 80°C pendant 1/2 heure. L'analyse chromatographique (G.P.C) montre que la réaction est terminée et qu'il s'est formé 25 % de produit de diaddition symétrique. On ajoute ensuite 0,120 partie d'éther monométhylique de l'hydroquinone, puis on coule à 80°C et en 10 mn, une solution de 74 parties de méthacrylate de t.butyl amino-éthyle (0,4 mole) dans 74 parties de méthyl isobutyl cétone. Le mélange est maintenu à 80°C pendant 1h30, puis refroidi. On obtient ainsi 875 parties d'une solution jaune ($S_1$) d'un monomère selon l'invention. Cette solution contient 33 % de matières séches et cristallise partiellement lors du stockage à froid.

EXEMPLE 2

Dans un réacteur d'une capacité de 250 parties en volume, équipé d'un agitateur, d'un thermomètre, d'un réfrigérant à reflux, d'une arrivée d'azote et d'un dispositif de chauffage, on charge 125 parties de la solution $S_1$. Après un balayage à l'azote en surface, la solution est chauffée à 90°C et on y ajoute alors 0,3 partie de peroxyde de lauroyle et 0,2 partie de perpivalate de t.butyle. On maintient ensuite la température à 90°C pendant 6heures en ajoutant la même charge d'initiateurs au bout de 2 et 4 heures. On observe la disparition complète du monomère en analyse chromatographique. On obtient après refroidissement 124 parties d'une solution ($S_2$) jaune brun d'homopolymère selon l'invention. Cette solution contient 33,1 % de matières sèches et 11,3 % de fluor.

EXEMPLE 3

Dans un réacteur d'une capacité de 500 parties en volume, équipé comme celui de l'exemple 1, on charge 17,4 parties de toluène-diisocyanate (0,1 mole), 88 parties de méthyl isobutyl cétone anhydre, et 0,1 partie de dibutyl dilaurate d'étain. L'atmosphère du réacteur est rendue inerte par un courant d'azote anhydre, puis la température est portée à 80°C. On coule alors goutte à goutte en 40 mn une solution, desséchée par distillation, de 36,4 parties de perfluoro-hexyl-éthanol (0,1 mole) dans 36,4 parties de méthyl isobutyl cétone. Dix minutes après la fin de la coulée, la réaction est terminée. On introduit alors en 5 mn une solution de 18,5 parties de méthacrylate de tertiobutylamino-éthyle (0,1 mole) dans 18,5 parties de méthyl isobutyl cétone anhydre, et maintient la température à 80°C pendant encore 15 mn. Puis on ajoute 72,3 parties de méthacrylate d'éthyl-2 hexyle et 74,4 parties de méthyl isobutyl cétone et porte la température de la solution à 90°C. La polymérisation est initiée par addition de 0,6 partie de peroxyde de lauroyle et 0,4 partie de perpivalate de t.butyle et entretenue par addition des mêmes quantités d'initiateurs toutes les deux heures. Au bout de 6 heures la solution est refroidie et filtrée. On obtient ainsi un copolymère selon l'invention à 50 % de monomère uréthanne-urée et 50 % de méthacrylate d'éthyl-2 hexyle sous forme d'une solution ($S_3$) visqueuse jaune ambrée qui contient 39 % de matière séche et 6,64 % de fluor.

EXEMPLE 4

Dans un réacteur identique à celui de l'exemple 2, on introduit 88,65 parties de solution ($S_1$) contenant 33 % de monomère uréthanneurée, 9 parties de méthacrylate de butyle, 11,35 parties d'un mélange de monomères polyfluorés de formule :

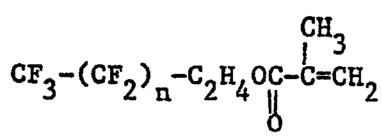

où n est égal à 5, 7, 9, 11, 13 et 15 dans des rapports en poids moyens et respectifs de 1 : 56 : 22 : 9 : 3 : 3, et 11,4 parties de méthyl isobutyl cétone. On chasse l'air du réacteur par un courant d'azote pendant 15 mn, puis on porte la température à 90°C. On ajoute alors 0,3 partie de peroxyde de lauroyle et 0,2 partie de perpivalate de t.butyle, cette addition étant renouvelée toutes les deux heures. Au bout de 6 heures, la polymérisation est terminée. La solution jaune brun est filtrée à 40°C. On obtient à froid un gel fluide ($S_4$) qui contient 42 % de matières non volatiles et 15,5 % de fluor.

EXEMPLE 5

Dans un réacteur identique à celui de l'exemple 2, on introduit 88,65 parties de solution ($S_1$) à 33 % de monomère uréthanne-urée, 9 parties de méthacrylate d'éthyl-2 hexyle, 16 parties d'un mélange de monomères polyfluorés de formule :

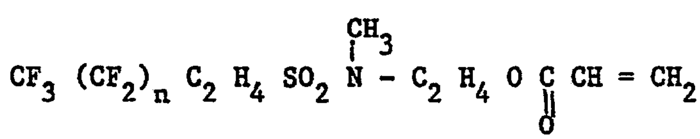

où n = 3, 5, 7, 9, 11, 13 et 15 dans les rapports en poids moyens et respectifs de 1 : 50 : 31 : 10 : 3 : 1 : 1, ainsi que 4,4 parties d'acétone et 7 parties de méthyl isobutyl cétone. La polymérisation est ensuite effectuée comme à l'exemple 4 et on obtient un gel ($S_5$) jaune brun dont le taux de matières non volatiles est de 42,6 % et le taux de fluor est de 13,5 %.

EXEMPLE 6

Dans un réacteur identique à celui de l'exemple 1 et selon le même mode opératoire, on ajoute goutte à goutte en une heure et à 80°C, une solution contenant 92,8 parties de perfluorooctyl-éthanol $C_8F_{17}C_2H_4OH$ (0,2 mole) et 92,8 parties de méthyl isobutyl cétone, dans une solution de 34,8 parties (0,2 mole) de toluène-diisocyanate (mélange à 80 % d'isomère 2,4), 0,2 partie de dibutyl dilaurate d'étain, et 254 parties de méthyl isobutyl cétone. On ajoute ensuite toujours à 80°C, 37 parties de méthacrylate de t.butylamino-éthanol (0,2 mole) dans 37 parties de méthyl-isobutyl cétone. Après 1h30 à 80°C la réaction est terminée et on obtient une solution ($S_6$) de monomère uréthanne-urée selon l'invention. Cette solution contient 30 % de matière séche et 11,78 % de fluor.

EXEMPLE 7

Dans un réacteur identique à celui de l'exemple 2, on introduit 83.3 parties de la solution ($S_6$), 18 parties de méthylisobutyl cétone, 25 parties de méthacrylate de butyle. Après avoir purgé le réacteur à l'azote, on porte la température à 90°C et ajoute 0,3 partie de peroxyde de lauroyle et 0,2 partie de perpivalate de t.butyle. Le mélange est chauffé pendant 6 heures à 90°C, tandis que la polymérisation est entretenue par addition de la même charge d'intitiateurs toutes les deux heures. La solution de copolymère obtenue ($S_7$) est homogène, mais opaque et visqueuse. Elle contient 39,3 % de matières non volatiles, et 7,7 % de fluor.

EXEMPLE 8

On procède comme à l'exemple 1. A un mélange de 17,4 parties de toluène-diisocyanate (0,1 mole), 0,1 partie de dibutyle dilaurate d'étain, et 140 parties de méthylisobutyl cétone, on ajoute goutte à goutte en une heure et à 80°C, une solution préalablement étêtée de 48,5 parties de sulfamido alcool fluoré de formule

$$C_6F_{13}C_2H_4\ SO_2N(CH_3)C_2H_4OH \qquad (0,1\ mole)$$

dans 37,7 parties de méthyl isobutyl cétone. Puis on ajoute 0,030 partie d'éther méthylique de l'hydroquinone et 18,5 parties de méthacrylate de t.butylamino-éthyle (0,1 mole), et maintient le mélange pendant encore 2 heures à 80°C. On obtient ainsi une solution ($S_8$) de monomère uréthanne-urée selon l'invention, contenant 30 % de matières séches et 8,8 % de fluor.

EXEMPLE 9

On opère comme à l'exemple 7, pour copolymériser 83,4 parties de solution ($S_8$) avec 25 parties de méthacrylate de stéaryle dans 17 parties de méthyl isobutyl cétone. La solution jaune brun obtenue ($S_9$) contient 39,5 % de matières non volatiles et 5,8 % de fluor.

EXEMPLE 10

On procède comme à l'exemple 8 en remplaçant le sulfamidoalcool fluoré par 48 parties de perfluorooctyl-thioéthanol $C_8F_{17}C_2H_4SH$ (0,1 mole). La solution ($S_{10}$) obtenue contient 30 % de matières non volatiles et 11,5 % de fluor.

EXEMPLE 11

Dans les mêmes conditions qu'à l'exemple 7, on copolymérise 83,4 parties de solution ($S_{10}$) avec 25 parties de méthacrylate d'éthyl-2 hexyle dans 17 parties de méthyl isobutyl cétone. La solution jaune brun ($S_{11}$) obtenue comprend 35,5 % de matières non volatiles et 6,8 % de fluor.

EXEMPLE 12

Selon le même mode opératoire qu'à l'exemple 7, on copolymérise 83,4 parties de solution ($S_8$), 9 parties de méthacrylate de stéaryle et 16 parties d'ester acrylique fluoré de formule :

$$CF_3-(CF_2)_nC_2H_4\ SO_2-N(CH_3)-C_2H_4OC(=O)-CH=CH_2$$

où n est égal à 3, 5, 7, 9, 11, 13 et 15 dans les rapports en poids moyens et respectifs de 1 : 50 : 31 : 10 : 3 : 1 : 1, dans 13 parties de méthyl isobutyl cétone et 4 parties d'acétone. On obtient une solution ($S_{12}$) jaune brun, non homogène à froid, contenant 40,4 % de matières non volatiles et 11,9 % de fluor.

EXEMPLE 13

13-a : Selon le même procédé qu'à l'exemple 7, on prépare un copolymère à base de 18,5 parties de méthacrylate de t.butylaminoéthyle (0,1 mole), 49,3 parties de méthacrylate de butyle et 55,5 parties d'un mélange de méthacrylates d'alcools fluorés de formule :

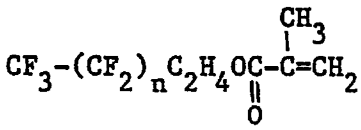

où n = 5, 7, 9, 11, 13, et 15 dans les rapports en poids moyens et respectifs de 1 : 56 : 22 : 9 : 3 : 3, dans 125 parties de méthyl isobutyl cétone.

13-b : En opérant comme à l'exemple 1 on ajoute en une heure à 80°C, 72,8 parties d'une solution à 50 % dans la méthyl isobutyl cétone de perfluorohexyl-éthanol $C_6F_{13}C_2H_4OH$ (0,1 mole), à 17,4 parties de toluène diisocyanate (à 80 % d'isomère-2,4) dans 110 parties de méthyl isobutyl cétone.

13-c : A l'isocyanate uréthanne obtenu en 13-b, on ajoute le polymère 13-a comportant des groupes pendants > N-H. Le mélange est ensuite chauffé pendant 2 heures à 100°C. On obtient ainsi une solution ($S_{13}$) d'un copolymère selon l'invention. Cette solution jaune brun, légérement visqueuse contient 38,4 % de matières non volatiles et 12,8 % de fluor.

EXEMPLE 14

On dilue les solutions de polymères $S_2$, $S_3$, $S_4$, $S_5$, $S_7$, $S_9$, $S_{11}$, $S_{12}$ et $S_{13}$ avec de la méthyl isobutyl cétone de façon à obtenir des solutions $S_{2d}$, $S_{3d}$, $S_{4d}$, $S_{5d}$, $S_{7d}$, $S_{9d}$, $S_{11d}$, $S_{12d}$ et $S_{13d}$ contenant chacune 0,15 % de fluor. Ces solutions diluées sont ensuite appliquées par pulvérisation sur un cuir vachette pleine fleur tanné vététal, à raison de 200g/m² et on laisse sécher pendant 4 heures à température ambiante avant de procéder aux tests suivants :

. TEST R.E. (résistance à l'eau) : consiste à mesurer le temps de pénétration d'une goutte d'eau déposée sur le cuir.

. TEST R.H. (résistance à l'huile) : consiste à mesurer le temps de pénétration d'une goute d'huile de vaseline déposée sur le cuir.

Le tableau suivant rassemble les résultats obtenus, comparativement au même cuir non traité.

| Solution de traitement | Hydrophobie RE | Oléophobie RH |
|---|---|---|
| Néant (cuir non traité) | moins de 30 secondes | moins de 10 secondes |
| $S_{2d}$ | 5,5 heures | 20 minutes |
| $S_{3d}$ | 6,5 heures | 50 minutes |
| $S_{4d}$ | 8 heures | plus de 24 heures |
| $S_{5d}$ | 8,5 heures | plus de 9 heures |
| $S_{7d}$ | 3,5 heures | 30 minutes |
| $S_{9d}$ | 7,5 heures | plus de 24 heures |
| $S_{11d}$ | 4,5 heures | 1 heure |
| $S_{12d}$ | 5,5 heures | plus de 24 heures |
| $S_{13d}$ | 1 heure | 3 heures |

EXEMPLE 15

Dans un réacteur de 250 parties en volume équipé d'un agitateur, d'un thermomètre, d'un réfrigérant à reflux, d'une ampoule de coulée et d'un système de chauffage (bain d'huile thermostaté), on introduit 92,5 parties de méthyl isobutyl cétone, 17,4 parties (0,1 mole) de toluène-diisocyanate (à 80 % d'isomère 2,4) et 0,1 partie de dibutyl dilaurate d'étain. La solution est chauffée sous atmosphère d'azote à 80°C, puis on ajoute goutte à goutte en une heure un mélange de 36,4 parties de perfluorohexyl-éthanol $C_6F_{13}C_2H_4OH$ (0,1 mole), 18,5 parties de méthacrylate de t.butylamino-éthyle (0,1 mole) et 54 parties de méthyl isobutyl cétone anhydre. La solution est maintenue encore 2 heures à 80°C et on obtient ainsi un mélange contenant en majorité le produit d'addition du méthacrylate de t.butylamino-éthyle en -2 et du perfluorohexyl-éthanol en -4 et -6, ainsi qu'une proportion molaire de 42 % de produits de diadditions symétriques. Ce mélange est porté à 90°C, puis on y ajoute 0,5 partie de peroxyde de lauroyle et 0,2 partie de perpivalate de t.butyle et renouvelle cette addition d'initiateurs aprés 2 et 4 heures de réaction. Au bout de 6 heures à 90°C, la réaction est terminée. La solution ($S_{15}$) obtenue est jaune brun, limpide ; elle contient 32,8 % de matières non volatiles et 11,2 % de fluor.

Cette solution est diluée à l'aide de méthyl isobutyl cétone jusqu'à une teneur en fluor de 0,15 %, puis appliquée à raison de 200 g/m² sur le même cuir qu'à l'exemple 14. Après séchage pendant une nuit à température ambiante, on obtient les résultats suivants :

RE = 7,5 heures et RH = 30 minutes.

EXEMPLE 16

Dans un réacteur identique à celui de l'exemple 1 on introduit 180 parties de trichlorotrifluoroéthane, 34,8 parties (0,2 mole) de toluène-diisocyanate (mélange à 80 % d'isomères -2,4 et 20 % d'isomère - 2,6), et 0,2 parties de dibutyldilaurate d'étain. Aprés avoir chassé l'air du réactuer par un courant d'azote anhydre et porté la température à 50°C (reflux du solvant), on ajoute goutte à goutte en une heure et quart une solution de 72,8 parties de perfluorohexyléthanol $C_6F_{13}C_2H_4OH$ dans 72,8 parties de trichlorotrifluoroéthane. On maintient ensuite le reflux pendant 1/2 heure et obtient une suspension blanchâtre dont l'analyse chromatographique montre qu'elle contient une proportion molaire d'environ 20 % de produit de diaddition-2,4 et 2,6. A la suspension blanchâtre obtenue, on ajoute 0,06 partie d'éther méthylique de l'hydroquinone et on coule ensuite en 15 minutes, 37 parties de méthacrylate de t.butyl amino-éthyle (0,2 mole), puis 40 parties de trichlorotrifluoroéthane. La réaction est légérement exothermique et on observe la dissolution du produit blanchâtre. Le reflux est maintenu pendant une heure, puis on ajoute 293 parties d'isopropanol et élimine le trichlorotrifluoro-éthane par distillation. On obtient une solution pratiquement incolore ($S_{16}$) contenant 33 % de matières non volatiles et 11,3 % de fluor.

EXEMPLE 17

Dans un réacteur de 500 parties en volume, équipé comme celui de l'exemple 2, on introduit 25 parties d'un mélange de monomères polyfluorés de formule :

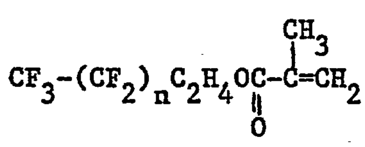

où n est égal à 5, 7, 9, 11, 13, 15 dans des rapports en poids moyens et respectifs de 1 : 56 : 22: 9 : 3 : 3 ; 20 parties de méthacrylate de méthoxytriéthylène glycol :

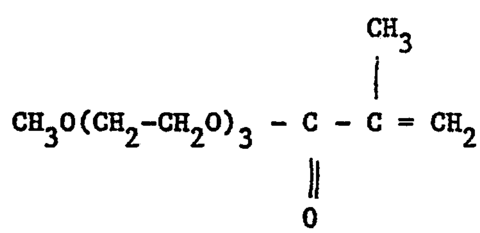

15,15 parties de la solution ($S_{16}$) et 50 parties d'alcool isopropylique. Aprés avoir chassé l'air du réacteur par un courant d'azote on chauffe le mélange à 82°C et ajoute 0,5 parties d'acide azo-4,4′ bis-(cyano-4 pentanoïque). On maintient le reflux pendant 5 heures en ajoutant chaque heure 0,1 partie de perpivalate de tertiobutyle. Puis on ajoute 50 parties d'un mélange 50/50 en poids isopropanoleau. Après refroidissement et filration on obtient une solution ($S_{17}$) qui contient 30,7 % de matières non volatiles et 10,5 % de fluor.

Une partie de cette solution est diluée jusqu'à 100 à l'aide d'un mélange 50/50 d'eau et d'isopropanol et la solution limpide obtenue (S $_{17d}$) est appliqué par pulvérisation sur deux types de cuir, à raison de 400 g/m$^2$. On laisse sécher pendant quatre heures à température ambiante avant de procéder à l'examen des performances selon la méthode indiquée précédemment.

| | R.E. | R.H |
|---|---|---|
| Cuir vachette pleine fleur tanné chrome Non-traité | inférieur à 30 secondes | inférieur à 1 minute |
| Même cuir traité avec $S_{17d}$ | 2,25 heures | plus de 9 heures |
| Cuir vachette pleine fleur tanné végétal Non-traité | inférieur à 30 secondes | inférieur à 10 secondes |
| Même cuir traité avec $S_{17d}$ | 3,25 heures | plus de 9 heures |

EXEMPLE 18

On opère comme à l'exemple 3 avec les modifications suivantes :

a/ le toluènediisocyanate est remplacé par 25 parties de diphénylméthane-diisocyanate-4,4′ (0,1 mole) et la quantité initiale de méthyl isobutyl cétone (88 parties) est portée à 132 parties,

b/ pour la polymérisation, on utilise 79,9 parties de méthacrylate d'éthyl-2 hexyle et seulement 53 parties de méthyl isobutyl cétone, l'initiation étant effectuée avec 1 partie de peroxyde de lauroyle et 0,6 partie de perpivalate de t.butyle.

Au bout de 6 heures, la solution est refroidie. On obtient ainsi un copolymère selon l'invention à 50 % de monomère uréthanneurée et 50 % de méthacrylate d'éthyl-2 hexyle sous forme d'une solution pâteuse ($S_{18}$) qui contient 40 % de matière sèche et 6 % de fluor.

Cette solution est diluée avec du perchloroéthylène pour obtenir une solution à 0,15 % de fluor qu'on applique à raison de 200 g/m$^2$ sur cuir vachette pleine fleur tanné végétal. Les résultats des tests sont les suivants :

R E : plus de 9 heures

R H : plus de 30 heures

**Revendications**

1. Monomères acryliques fluorés, caractérisés en ce qu'ils répondent à la formule générale:

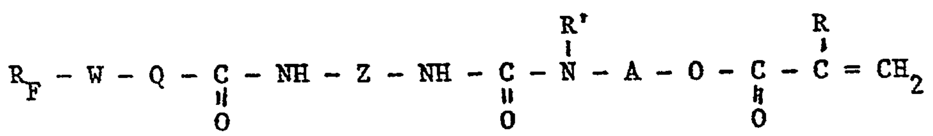
(I)

dans laquelle:

$R_F$ représente un radical perfluoroalkyle à chaîne droite ou ramifiée,

R représente un atome d'hydrogène ou un radical méthyle,

R′ représente un radical alkyle ou cycloalkyle ou –NR′– représente un radical pipérazinylène-1,4,

W–Q représente un enchaînement bivalent choisi parmi ceux de formules:

$$-(CH_2)_p-O-$$
$$-SO_2N(R'')-(CH_2)_q-O-$$
$$-(CH_2)_p-SO_2N(R'')-(CH_2)_q-O-$$
$$-(CH_2)_p-O-(CH_2)_q-O-$$
$$-(CH_2)_p-S-(CH_2)_q-O-$$
$$-(CH_2)_p-(OCH_2CH_2)_q-O-$$
$$-(CH_2)_p-SO_2-(CH_2)_q-O-$$
$$-C(=O)-N(R'')-(CH_2)_p-O-$$
$$-C(=O)-O-(CH_2)_p-O-$$
$$-CH=CH-(CH_2)_p-O-$$
$$-(CH_2)_pS-$$
$$-(CH_2)_pNH-$$
$$-SO_2-N(R'')-(CH_2)_qNH-$$
$$-SO_2-N(R'')-(CH_2)_q-N(CH_3)-$$

R″ désigne un atome d'hydrogène ou un radical alkyle,
p et q, identiques ou différents, représentent chacun un nombre entier allant de 1 à 20,
Z représente un enchaînement bivalent aliphatique, cycloaliphatique ou aromatique, et
A représente un groupe alkylène de 2 ou 3 atomes de carbone.

2. Monomères selon la revendication 1, dans lesquels A est le groupement éthylène, R′ le radical tertiobutyle et Z le groupement tolylène-2,4 et/ou -2,6.

3. Monomères selon la revendication 1 ou 2, dans lesquels –W–Q– est un enchaînement
$-CH_2CH_2O-$, $-CH_2CH_2S-$
ou $-CH_2CH_2-SO_2N(R'')-CH_2CH_2O-$.

4. Monomères selon l'une des revendications 1 à 3, dans lesquels $R_F$ est un radical perfluoroalkyle linéaire contenant 6, 8 ou 10 atomes de carbone.

5. Procédé de préparation des monomères selon l'une des revendications 1 à 4, caractérisé en ce qu'on fait réagir sur un diisocyanate de formule O=C=N–Z–N=C=O, des quantités équimolaires d'un composé polyfluoré de formule $R_F$–W–Q–H et d'un ester de formule R′NH–A–OCO–C(R)=$CH_2$, la réaction étant effectuée au sein d'un solvant organique, à une température de 30 à 90°C et sous atmosphère inerte.

6. Procédé selon la revendication 5, dans lequel on fait d'abord réagir le composé polyfluoré $R_F$–W–Q–H, puis l'ester R′NH–A–OCO–C(R)=$CH_2$.

7. Polymères contenant de 10 à 100% en poids de motifs de formule:

$$R_F-W-Q-C(=O)-NH-Z-NH-C(=O)-N(R')-A-O-C(=O)-C(R)(-)-CH_2-$$

obtenus par homopolymérisation des monomères selon l'une des revendications 1 à 4, par copolymérisation desdits monomères avec d'autres monomères fluorés ou non, ou par greffage d'un isocyanate-uréthanne fluoré de formule:

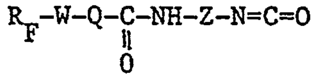

(XII)

sur un homopolymère d'un ester de formule R'NH–A–O–CO–C(R)=$CH_2$ ou un copolymère d'un tel ester avec d'autre monomères fluorés ou non, les symboles $R_F$, R, R', W–Q, Z et A ayant les mêmes significations que dans les revendications 1 à 4.

8. Copolymères selon la revendication 7, dans lesquels le ou les comonomères sont choisis parmi les acrylates ou méthacrylate d'alkyle ou de cellosolve, les composés fluorés de formule:

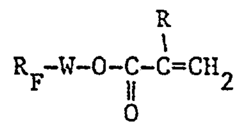

et leurs mélanges,
W représente un enchaînement bivalent choisi parmi ceux de formules:

$-(CH_2)_p-$

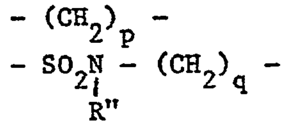

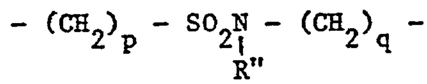

$-(CH_2)_p-O-(CH_2)_q-$

$-(CH_2)_p-S-(CH_2)_q-$

$-(CH_2)_p-(OCH_2CH_2)_q-$

$-(CH_2)_p-SO_2-(CH_2)_q-$

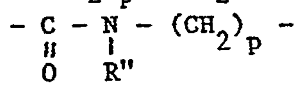

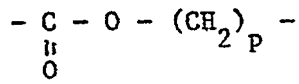

$-CH=CH-(CH_2)_p-$

les symboles $R_F$, R, R'', p et q ayant les mêmes significations que dans la revendication 1.

9. Application des polymères selon la revendication 7 ou 8 à l'oléofugation et à l'hydrofugation de substrats divers, en particulier des cuirs.

10. Matériaux et articles traités au moyen d'un polymère selon la revendication 7 ou 8.

**Patentansprüche**

1. Fluorierte acrylische Monomere, dadurch gekennzeichnet, daß sie der allgemeinen Formel entsprechen:

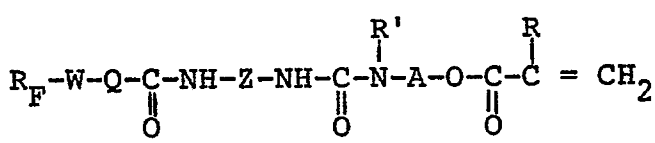

(I)

in der
$R_F$ einen Perfluoralkylrest mit gerader oder verzweigter Kette darstellt,
R ein Wasserstoffatom oder einen Methylrest darstellt,
R′ einen Alkylrest oder Cycloalkylrest darstellt oder NR′ einen 1,4-Piperazinylenrest darstellt,
W–Q einen bivalenten Kettenabschnitt darstellt, ausgewählt aus denjenigen der Formeln:

$$-(CH_2)_p-O-$$
$$-SO_2N(R'')-(CH_2)_q-O-$$
$$-(CH_2)_p-SO_2N(R'')-(CH_2)_q-O-$$
$$-(CH_2)_p-O-(CH_2)_q-O-$$
$$-(CH_2)_p-S-(CH_2)_q-O-$$
$$-(CH_2)_p-(OCH_2CH_2)_q-O-$$
$$-(CH_2)_p-SO_2-(CH_2)_q-O-$$
$$-C(=O)-N(R'')-(CH_2)_p-O-$$
$$-C(=O)-O-(CH_2)_p-O-$$
$$-CH=CH-(CH_2)_p-O-$$
$$-(CH_2)_pS-$$
$$-(CH_2)_pNH-$$
$$-SO_2-N(R'')-(CH_2)_qNH-$$
$$-SO_2-N(R'')-(CH_2)_q-N(CH_3)-$$

wobei
R″ ein Wasserstoffatom oder einen Alkylrest darstellt,
p und q identisch oder verschieden sind und jeweils eine ganze Zahl von 1 bis 20 darstellen,
Z einen bivalenten aliphatischen, cycloaliphatischen oder aromatischen Kettenabschnitt darstellt und
A eine Alkylengruppe mit 2 oder 3 Kohlenstoffatomen darstellt.

2. Monomere nach Anspruch 1, bei denen A eine Ethylengruppierung, R′ ein Tertiärbutylrest und Z eine 2,4-Tolylen und/oder 2,6-Tolylengruppierung ist.

3. Monomere nach Anspruch 1 oder 2, bei denen –W–Q– einen Kettenabschnitt

$-CH_2CH_2O-$, $-CH_2CH_2S-$ oder
$-CH_2CH_2-SO_2N(R'')-CH_2CH_2O-$

darstellt.

4. Monomere nach einem der Ansprüche 1 bis 3, in denen $R_F$ ein linearer Perfluoralkylrest mit 6, 8 oder 10 Kohlenstoffatomen ist.

5. Verfahren zur Herstellung von Monomeren gemäß einem der Ansprüche 1 bis 4, dadurch gekennzeichnet, daß man mit einem Diisocyanat der Formel O=C=N–Z–N=C=O äquimolare Mengen einer polyfluorierten Verbindung der Formel $R_F$–W–Q–H und eines Esters der Formel R′NH–A–OCO–C(R)=$CH_2$ reagieren läßt, wobei die Reaktion in einem organischen Lösungsmittel bei einer Temperatur von 30 bis 90°C und unter Inertatmosphäre durchgeführt wird.

6. Verfahren nach Anspruch 5, bei dem man zunächst die polyfluorierte Verbindung $R_F$–W–Q–H und dann den Ester R'NH–A–OCO–C(R)=$CH_2$ reagieren läßt.

7. Polymere enthaltend 10 bis 100 Gewichtsprozent Baueinheiten der Formel:

$$R_F-W-Q-C(=O)-NH-Z-NH-C(=O)-N(R')-A-O-C(=O)-C(R)(-)-CH_2-$$

erhalten durch Homopolymerisation von Monomeren gemäß einem der Ansprüche 1 bis 4, durch Copolymerisation dieser Monomere mit anderen fluorierten oder nicht fluorierten Monomeren oder durch Aufpfropfung eines fluorierten Isocyanat-Urethans der Formel

$$R_F-W-Q-C(=O)-NH-Z-N=C=O \qquad (XII)$$

auf ein Homopolymeres eines Esters der Formel R'NH–A–O–CO–C(R)=$CH_2$ oder ein Copolymer eines solchen Esters mit anderen fluorierten oder nicht fluorierten Monomeren, wobei die Symbole $R_F$, R, R', W–Q, Z und A die gleiche Bedeutung haben wie in den Ansprüchen 1 bis 4.

8. Copolymere nach Anspruch 7, in denen das oder die Comonomeren ausgewählt werden aus den Alkylacrylaten oder -methacrylaten oder Cellosolveacrylaten oder -methacrylaten, den fluorierten Verbindungen der Formel:

$$R_F-W-O-C(=O)-C(R)=CH_2$$

und deren Gemischen, wobei
W einen bivalenten Kettenabschnitt ausgewählt aus denjenigen der Formeln:

$$-(CH_2)_p-$$
$$-SO_2N(R'')-(CH_2)_q-$$
$$-(CH_2)_p-SO_2N(R'')-(CH_2)_q-$$
$$-(CH_2)_p-O-(CH_2)_q-$$
$$-(CH_2)_p-S-(CH_2)_q-$$
$$-(CH_2)_p-(OCH_2CH_2)_q-$$
$$-(CH_2)_p-SO_2-(CH_2)_q-$$
$$-C(=O)-N(R'')-(CH_2)_p-$$
$$-C(=O)-O-(CH_2)_p-$$
$$-CH=CH-(CH_2)_p-$$

darstellt, in denen die Symbole $R_F$, R, R'', p und q die gleiche Bedeutung haben wie in Anspruch 1.

9. Verwendung von Polymeren nach Anspruch 7 oder 8 zur Oleophobierung und Hydrophobierung unterschiedlicher Substrate, insbesondere von Leder.

10. Materialien und Artikel, die mittels eines Polymers gemäß Anspruch 7 oder 8 behandelt wurden.

## Claims

1. Fluorinated acrylic monomers characterized in that they correspond to the general formula:

$$R_F - W - Q - C(=O) - NH - Z - NH - C(=O) - N(R') - A - O - C(=O) - C(R) = CH_2 \qquad (I)$$

in which:
$R_F$ denotes a straight or branched chain perfluoroalkyl radical,
R denotes a hydrogen atom or a methyl radical,
R′ denotes an alkyl or cycloalkyl radical or –NR′– denotes a 1,4-piperazinylene radical,
W–Q denotes a divalent chain sequence chosen from those of formulae:

$$-(CH_2)_p-O-$$
$$-SO_2N(R'')-(CH_2)_q-O-$$
$$-(CH_2)_p-SO_2N(R'')-(CH_2)_q-O-$$
$$-(CH_2)_p-O-(CH_2)_q-O-$$
$$-(CH_2)_p-S-(CH_2)_q-O-$$
$$-(CH_2)_p-(OCH_2CH_2)_q-O-$$
$$-(CH_2)_p-SO_2-(CH_2)_q-O-$$
$$-C(=O)-N(R'')-(CH_2)_p-O-$$
$$-C(=O)-O-(CH_2)_p-O-$$
$$-CH=CH-(CH_2)_p-O-$$
$$-(CH_2)_pS-$$
$$-(CH_2)_pNH-$$
$$-SO_2-N(R'')-(CH_2)_qNH-$$
$$-SO_2-N(R'')-(CH_2)_q-N(CH_3)-$$

R″ denotes a hydrogen atom or an alkyl radical, each of p and q, which are identical or different, denotes an integer ranging from 1 to 20,
Z denotes an aliphatic, cycloaliphatic or aromatic divalent chain sequence, and
A denotes an alkylene group of 2 or 3 carbon atoms.

2. Monomers according to Claim 1, in which A is the ethylene group, R′ the tert-butyl radical and Z the 2,4- and/or 2,6-tolylene group.

3. Monomers according to Claim 1 or 2, in which –W–Q– is a chain sequence
$-CH_2CH_2O-$, $-CH_2CH_2S-$
or $-CH_2CH_2-SO_2N(R'')-CH_2CH_2O-$.

4. Monomers according to one of Claims 1 to 3, in which $R_F$ is a linear perfluoroalkyl radical containing 6, 8 or 10 carbon atoms.

5. Process for the preparation of the monomers according to one of Claims 1 to 4, characterized in that a diisocyanate of formula O=C=N–Z–N=C=O is reacted with equimolar quanities of a polyfluorinated compound of formula $R_F$–W–Q–H and an ester of formula R'NH–A–OCO–C(R)=$CH_2$, the reaction being carried out in an organic solvent at a temperature of 30 to 90°C and under an inert atmosphere.

6. Process according to Claim 5, in which the polyfluorinated compound $R_F$–W–Q–H is reacted first of all, followed by the ester R'NH–A–OCO–C(R)=$CH_2$.

7. Polymers containing from 10 to 100% by weight of units of formula:

$$R_F - W - Q - \underset{\overset{\|}{O}}{C} - NH - Z - NH - \underset{\overset{\|}{O}}{C} - \overset{\overset{R'}{|}}{N} - A - O - \underset{\overset{\|}{O}}{C} - \underset{\backslash}{\overset{\overset{R}{|}}{C}} - CH_2 —$$

which are obtained by homopolymerization of the monomers according to one of Claims 1 to 4, by copolymerization of the said monomers with other monomers, fluorinated or otherwise, or by grafting a fluorinated isocyanate-urethane of formula:

$$R_F\text{-}W\text{-}Q\text{-}\underset{\overset{\|}{O}}{C}\text{-}NH\text{-}Z\text{-}N{=}C{=}O \qquad (XII)$$

onto a homopolymer of an ester of formula R'NH–A–O–CO–C(R)=$CH_2$ or a copolymer of such an ester with other monomers, fluorinated or otherwise, the symbols $R_F$, R, R', W–Q, Z and A having the same meanings as in claims 1 to 4.

8. Copolymers according to Claim 7, in which the comonomer(s) is (are) chosen from alkyl or cellosolve acrylates or methacrylate, the fluorinated compounds of formula:

$$R_F\text{-}W\text{-}O\text{-}\underset{\overset{\|}{O}}{C}\text{-}\overset{\overset{R}{\backslash}}{C}{=}CH_2$$

and mixtures thereof,

W denotes a divalent chain sequence chosen from those of formulae:

$$-(CH_2)_p-$$
$$-SO_2\underset{\underset{R''}{|}}{N}-(CH_2)_q-$$
$$-(CH_2)_p-SO_2\underset{\underset{R''}{|}}{N}-(CH_2)_q-$$
$$-(CH_2)_p-O-(CH_2)_q-$$
$$-(CH_2)_p-S-(CH_2)_q-$$
$$-(CH_2)_p-(OCH_2CH_2)_q-$$
$$-(CH_2)_p-SO_2-(CH_2)_q-$$
$$-\underset{\overset{\|}{O}}{C}-\underset{\underset{R''}{|}}{N}-(CH_2)_p-$$
$$-\underset{\overset{\|}{O}}{C}-O-(CH_2)_p-$$
$$-CH=CH-(CH_2)_p-$$

the symbols $R_F$, R, R″, p and q having the same meanings as in Claim 1.

9. Application of the polymers according to Claim 7 or 8 to the imparting of oil-repellency and water-repellency to various substrates, in particular to leathers.

10. Materials and articles treated by means of a polymer according to Claim 7 or 8.